(19) **Europäisches Patentamt / European Patent Office / Office européen des brevets**

(11) **EP 4 778 517 A1**

(12) **EUROPEAN PATENT APPLICATION**
published in accordance with Art. 153(4) EPC

(43) Date of publication:
22.07.2026 Bulletin 2026/30

(21) Application number: 24865236.4

(22) Date of filing: 28.08.2024

(51) International Patent Classification (IPC):
A61K 31/122 (2006.01)    A61K 9/14 (2006.01)
A61K 31/19 (2006.01)    A61K 31/455 (2006.01)
A61K 47/10 (2017.01)    A61K 47/14 (2017.01)
A61K 47/22 (2006.01)    A61K 47/26 (2006.01)
A61K 47/34 (2017.01)

(52) Cooperative Patent Classification (CPC):
A61K 9/14; A61K 31/122; A61K 31/19;
A61K 31/455; A61K 47/10; A61K 47/14;
A61K 47/22; A61K 47/26; A61K 47/34

(86) International application number:
PCT/JP2024/030631

(87) International publication number:
WO 2025/057734 (20.03.2025 Gazette 2025/12)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 13.09.2023 JP 2023148348

(71) Applicant: KANEKA CORPORATION
Osaka-shi, Osaka 530-8288 (JP)

(72) Inventors:
• UEDA Takahiro
  Tokyo 107-6028 (JP)
• TSUDA Satoru
  Osaka-shi, Osaka 530-8288 (JP)
• YAMAGUCHI Takao
  Takasago-shi, Hyogo 676-8688 (JP)

(74) Representative: Hoffmann Eitle
Patent- und Rechtsanwälte PartmbB
Arabellastraße 30
81925 München (DE)

(54) **SOLID COMPOSITION CONTAINING CO-CRYSTAL OF REDUCED COENZYME Q10**

(57) The present specification discloses a means capable of controlling the decrease of reduced coenzyme Q10 due to its oxidation during storage of a solid composition containing a co-crystal of reduced coenzyme Q10. One or more embodiments of the present invention relate to a solid composition containing a co-crystal of reduced coenzyme Q10, an emulsifier, and an antioxidant. Another one or more embodiments of the present invention relate to a method for producing a particulate solid composition containing a co-crystal of reduced coenzyme Q10, an emulsifier, an antioxidant, and a binder, the method comprising granulating a mixture containing the co-crystal of reduced coenzyme Q10, the emulsifier, the antioxidant, the binder, and a liquid binder.

**Description**

Technical Field

One or more embodiments of the present invention relate to a solid composition containing a co-crystal of reduced coenzyme Q10.
Another one or more embodiments of the present invention relate to a method for producing a particulate solid composition containing a co-crystal of reduced coenzyme Q10.

Background Art

Coenzyme Q is an essential component widely distributed in living organisms from bacteria to mammals, and is known as a constituent of the mitochondrial electron transfer system in cells in living organisms. In humans, the major form of coenzyme Q is coenzyme Q10, the side chain of which has ten repeating structures. In the living body, approximately 40% to 90% of the coenzyme Q10 is generally present in reduced form. The physiological activity of coenzyme Q includes activation of energy production by mitochondrial activation, activation of cardiac function, stabilization of cell membranes, and protection of cells by antioxidant activity.
While coenzyme Q10 currently produced and sold is, in large part, oxidized coenzyme Q10, reduced coenzyme Q10 (hereinafter sometimes referred to as "QH") which exhibits higher oral absorbability than oxidized coenzyme Q10 has also been commercially available and has come to be used in recent years.
Reduced coenzyme Q10 is easily oxidized, and therefore has the problems of high storage costs and limited scope of application to commercial forms. In particular, there is a demand for a solid composition that contains reduced coenzyme Q10, has excellent oxidation stability, and can be applied to a form such as a granule, hard capsule, or tablet.
Patent Literature 1 discloses that a solid composition containing reduced coenzyme Q10 is coated with a coating material such as shellac, gelatin, or gum arabic to be thereby stabilized.
Patent Literature 2 discloses a particulate composition in which an oily component containing reduced coenzyme Q10 is polydispersed in domain form in a matrix containing a water-soluble excipient and a water-soluble ascorbic acid.
Patent Literature 3 discloses that crystal polymorphism is found in reduced coenzyme Q10, and that a newly appearing crystalline form (this crystal is hereinafter referred to as "reduced coenzyme Q10 Form II crystal", "QH Form II crystal", or "Form II crystal") is much more stable than the conventional reduced coenzyme Q10 (this crystal is hereinafter referred to as a "reduced coenzyme Q10 Form I crystal", "QH Form I crystal", or "Form I crystal"), and also has other excellent physical properties.
Patent Literature 4 describes a reduced coenzyme Q10 composition that is suited for an inexpensive simple formulation capable of meeting market demand, has good oxidation stability, and is in a solid state such as a powder at room temperature, wherein the composition contains reduced coenzyme Q10, an organic acid, and a carbonate containing a sodium cation and/or a calcium cation, and is solid at 25°C. Patent Literature 4 discloses that the reduced coenzyme Q10 is preferably a Form II crystal. Patent Literature 4 discloses that, to improve the oxidation stability of the reduced coenzyme Q10 in the composition, it is essential to incorporate a carbonate containing a sodium cation and/or a calcium cation.
Also, Patent Literature 5 describes that reduced coenzyme Q10 is able to form a co-crystal with nicotinamide and that such co-crystal exhibits higher oxidation stability than a conventional reduced coenzyme Q10 crystal.
Patent Literature 6 describes a novel granule that can adsorb and solidify a large amount of a hardly soluble functional substance, and allow the functional substance to have excellent elution ability in water, wherein the granule contains: a support containing a porous calcium silicate; a functional substance and a nonionic surfactant that are adsorbed onto the support; and a binder, and wherein the functional substance is at least one selected from the group consisting of: a hardly soluble liquid; a hardly soluble solid having a low melting point; and a crude-drug extract containing a hardly soluble active ingredient. In Patent Literature 5, coenzyme Q10 is described as a specific example of a functional substance.
Patent Literature 7 describes a method for producing a particulate product containing an emulsifier by tumbling granulation, but discloses that granulation using an emulsifier having a melting point lower than 25°C, i.e., an emulsifier that is liquid at ordinary temperature, causes the granulated product to form lumps, often failing to give a desired granulated product.

Citation List

Patent Literature

Patent Literature 1: WO 2006/075502
Patent Literature 2: WO 2008/129980
Patent Literature 3: WO 2012/176842
Patent Literature 4: WO 2015/122531
Patent Literature 5: CN 113024362 A
Patent Literature 6: JP 2010-189337 A
Patent Literature 7: JP S60-221078 A

Summary of Invention

Technical Problem

However, such a solid composition containing reduced coenzyme Q10 as conventionally disclosed is produced by a complicated production method, and there is still need for improvement in the oxidation stability of reduced coenzyme Q10.

Production of the solid composition in Patent Literature 1 requires a step of spraying a coating material solution and a subsequent step of drying. In addition, production of the particulate composition in Patent Literature 2 requires: a step of preparing an oil-in-water emulsified product with an aqueous solution of a water-soluble excipient such as gum arabic and with an oily component containing reduced coenzyme Q10; and a step of removing water from droplets of the emulsified composition by spray-drying or oil-heating to form the composition into particles.

When a coating material such as a water-soluble excipient is used to give gas barrier properties to a solid composition containing reduced coenzyme Q10, the solid composition needs to be completely coated, which leads to various problems such as limited production method, higher costs, larger size of the solid composition, or less amount of reduced coenzyme Q10 contained in the solid composition. Furthermore, the solid composition containing reduced coenzyme Q10 produced by the above-described method will crack when subjected to pressure, resulting in a loss of gas barrier properties, and is therefore not suitable as a raw material for further formulation such as tableting.

The present inventors had examined the reduced coenzyme Q10 Form II crystal in Patent Literature 3 and the co-crystal of reduced coenzyme Q10 in Patent Literature 5. As a result, it was revealed that such crystals would exhibit higher oxidation stability than conventional crystals of reduced coenzyme Q10 but would be desired to be further stabilized in order to be formed into hard capsules or tablets. In addition, the compositions in Patent Literature 4 and Patent Literature 6 still have needs for improvement in stabilizing reduced coenzyme Q10, and applications of such compositions would be limited because they would require carbonate and porous calcium silicate as essential components.

The present specification discloses a means capable of controlling the decrease of reduced coenzyme Q10 due to its oxidation during storage of a solid composition containing the reduced coenzyme Q10.

Solution to Problem

The present inventors have completed each of the below-described embodiments of the present invention through the new discovery that, a solid composition containing a co-crystal of reduced coenzyme Q10 together with an emulsifier and an antioxidant, such as a solid composition obtained by granulating a co-crystal of reduced coenzyme Q10 and a solid antioxidant in the presence of a specific emulsifier using a granulation method with pressure or a fluidized-bed granulation method, allows to control the decrease of the reduced coenzyme Q10 in the solid composition during storage due to oxidation.

(1) A solid composition containing a co-crystal of reduced coenzyme Q10, an emulsifier, and an antioxidant.
(2) The solid composition according to (1), wherein the emulsifier is in the form of liquid, sol, gel, or soft solid at 50°C.
(3) The solid composition according to (1) or (2), wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.
(4) The solid composition according to any one of (1) to (3), wherein an amount of the emulsifier contained in the solid composition is 1% by weight or more and 99% by weight or less.
(5) The solid composition according to any one of (1) to (4), wherein the antioxidant is one or more selected from the group consisting of ascorbic acid, ascorbic acid salts, erythorbic acid, and erythorbic acid salts.
(6) The solid composition according to any one of (1) to (5), wherein the co-crystal of reduced coenzyme Q10 is a co-crystal of reduced coenzyme Q10 with benzoic acids or a co-crystal of reduced coenzyme Q10 with nicotinamide.
(7) The solid composition according to any one of (1) to (6), wherein 70% by weight or more of the reduced coenzyme Q10 is contained in the solid composition in the form of the co-crystal of reduced coenzyme Q10.

(8) The solid composition according to any one of (1) to (7), wherein a residual rate of the reduced coenzyme Q10 after the solid composition is stored under the environment of 40°C and 75%RH in an open state for 30 days is 93% or more.

(9) The solid composition according to any one of (1) to (8), further containing a binder.

(10) The solid composition according to (9), wherein the solid composition is a particulate solid composition.

(11) The solid composition according to any one of (1) to (10), wherein the solid composition is free of a carbonate containing a sodium cation and/or a calcium cation.

(12) The solid composition according to any one of (1) to (11), wherein the solid composition is free of porous calcium silicate.

(13) A method for producing a particulate solid composition containing a co-crystal of reduced coenzyme Q10, an emulsifier, an antioxidant, and a binder,

the method comprising granulating a mixture containing the co-crystal of reduced coenzyme Q10, the emulsifier, the antioxidant, the binder, and a liquid binder.

(14) The method according to (13), wherein the granulating is performed by an extrusion granulation method, a high-shear granulation method, or a fluidized-bed granulation method.

The present specification encompasses the disclosure of Japanese Patent Application No. 2023-148348 that serves as a basis for the priority of the present application.

Advantageous Effects of Invention

In the solid composition according to one or more embodiments of the present invention, the decrease of reduced coenzyme Q10 due to its oxidation during storage can be controlled.

The method according to another one or more embodiments of the present invention can produce a particulate solid composition containing a co-crystal of reduced coenzyme Q10 and having excellent storage stability.

In addition, the method according to one or more embodiments of the present invention can produce a particulate solid composition containing a co-crystal of reduced coenzyme Q10 and having excellent handling properties at a higher yield.

Description of Embodiments

Hereinafter, the present invention will be described in detail.

<Reduced Coenzyme Q10>

The reduced coenzyme Q10 herein may partially include oxidized coenzyme Q10 as long as the reduced coenzyme Q10 is included as a main component. The main component herein means that it is included in a percentage of, for example, 50% by weight or more, typically 60% by weight or more, preferably 70% by weight or more, more preferably 80% by weight or more, still more preferably 90% by weight or more, particularly preferably 95% by weight or more, and particularly 98% by weight or more. The above-described percentages are the percentages of the reduced coenzyme Q10 to the total weight of coenzyme Q10.

As described above, the reduced coenzyme Q10 encompasses two forms of crystal polymorphisms, i.e., Form I and Form II. Specifically, the crystalline form of reduced coenzyme Q10 having a melting point around 48°C and showing characteristic peaks at diffraction angles ($2\theta \pm 0.2°$) of 3.1°, 18.7°, 19.0°, 20.2°, and 23.0° in powder X-ray (Cu-K$\alpha$) diffraction is a Form I crystal, whereas the crystalline form of reduced coenzyme Q10 having a melting point around 52°C and showing characteristic peaks at diffraction angles ($2\theta \pm 0.2°$) of 11.5°, 18.2°, 19.3°, 22.3°, 23.0°, and 33.3° in powder X-ray (Cu-K$\alpha$) diffraction is a Form II crystal.

<Co-crystal of Reduced Coenzyme Q10>

The term "co-crystal" used herein refers to a crystalline solid in which two or more different unionized compounds are alternately disposed in a space to form a crystal lattice.

The term "co-crystal of reduced coenzyme Q10" refers to a co-crystal in which one compound is reduced coenzyme Q10.

Examples of other compounds that form co-crystals with reduced coenzyme Q10 in a co-crystal of reduced coenzyme Q10 include polyphenols, benzoic acids, carboxylic acids, nitrogen-containing heterocyclic compounds, and amino acids.

Examples of polyphenols include, but are not particularly limited to, catechin, epigallocatechin, tannin, rutin, isoflavone, resveratrol, curcumin, luteolin, and naringenin, with catechin, resveratrol, luteolin, naringenin, and the like being preferable.

Examples of benzoic acids include, but are not particularly limited to, benzoic acid, 2-hydroxybenzoic acid, 3-hydroxybenzoic acid, 4-hydroxybenzoic acid, 2,4-dihydroxybenzoic acid, 2,5-dihydroxybenzoic acid, 3,4-dihydroxybenzoic acid, and 3,5-dihydroxybenzoic acid, with benzoic acid, 3-hydroxybenzoic acid, 2,5-dihydroxybenzoic acid, 3,4-dihydroxybenzoic acid, 3,5-dihydroxybenzoic acid, and the like being preferable.

Examples of carboxylic acids include, but are not particularly limited to, tartaric acid, malonic acid, succinic acid, malic acid, fumaric acid, stearic acid, palmitic acid, ferulic acid, and caffeic acid.

Examples of nitrogen-containing heterocyclic compounds include, but are not particularly limited to, piperazine, pipecolic acid, nicotinic acid, nicotinamide, isonicotinamide, and saccharin, with nicotinamide being preferable.

An amino acid may be in the D, L, or racemic form. Examples of amino acids include, but are not particularly limited to, glycine, alanine, arginine, leucine, isoleucine, lysine, valine, cysteine, histidine, methionine, phenylalanine, serine, threonine, tryptophan, tyrosine, aspartic acid, glutamic acid, N-methylglycine, $\gamma$-aminobutyric acid, proline, betaine, and carnitine, with lysine, valine, N-methylglycine, $\gamma$-aminobutyric acid, proline, betaine, and the like being preferable.

The co-crystal of reduced coenzyme Q10 is preferably a co-crystal of reduced coenzyme Q10 with the benzoic acids or a co-crystal of reduced coenzyme Q10 with nicotinamide, and it is particularly preferably a co-crystal of reduced coenzyme Q10 with nicotinamide.

One or more embodiments of the present invention are characterized by the use of the co-crystal of reduced coenzyme Q10. By preparing a co-crystal of reduced coenzyme Q10 in the form of a solid composition containing the co-crystal together with an emulsifier and an antioxidant, the oxidation stability of the reduced coenzyme Q10 can further be enhanced compared with that of the co-crystal by itself.

<Emulsifier>

Herein, the emulsifier is not limited to any kind. For example, an emulsifier having an HLB of 1 or more and 17 or less, preferably 2 or more and 16 or less, may be used. Two or more kinds of emulsifiers may be used in combination.

Among the above-described emulsifiers, an emulsifier that is not in the form of powder or flakes is preferable from the viewpoint of the effect of controlling the oxidation of reduced coenzyme Q10. Examples of the emulsifier that is not in the form of powder or flakes include emulsifiers that are in the form of liquid, sol, gel, or soft solid. An emulsifier that is in the form of liquid, viscous liquid, sticky liquid, paste, pellets, waxy lump, wax, soft solid, or semisolid is more preferable. The emulsifier is preferably in the above-described form at 50°C, most preferably in the above-described form at 25°C.

Examples of the emulsifier that is not in the form of powder or flakes include an emulsifier having a melting starting point of 50°C or less, preferably 40°C or less, more preferably 25°C or less, as measured with a differential scanning calorimeter (DSC) set at a heating rate of 1°C/min or more and 20°C/min or less.

From another viewpoint, the emulsifier may be, for example, an emulsifier having a viscosity of 150,000 mPa·s or less, preferably 100,000 mPa·s or less, 50,000 mPa·s or less, more preferably 30,000 mPa·s or less, most preferably 25,000 mPa·s or less, as measured using a Brookfield type viscometer at a rotational rate of 10 rpm at a sample temperature of 50°C. The lower limit of the viscosity is not particularly limited, as long as it is greater than 0 mPa·s, and is more preferably 1 mPa·s or more, more preferably 5 mPa·s or more, most preferably 10 mPa·s or more.

In one or more embodiments of the present invention, a particulate solid composition obtained using the above-described emulsifier that is not in the form of powder or flakes retains good handling properties (dischargeability from a granulator, mesh passability, conveyability, and the like) contrary to the suggestion of Patent Literature 6 or the like, resulting in the particulate solid composition with both oxidative stability of the reduced coenzyme Q10 contained and good handling properties.

Specific examples of the emulsifier include one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

The number of glycerol units in the polyglycerol may be 2 or more, and is preferably 2 or more and 10 or less. Examples include diglycerol, triglycerol, tetraglycerol, pentaglycerol, hexaglycerol, and decaglycerol.

The number of oxyethylene units in the polyoxyethylene sorbitan may be 2 or more, preferably 10 or more and 30 or less, more preferably 15 or more and 25 or less.

The number of propylene glycol units in the polypropylene glycol may be 2 or more, and is preferably 2 or more and 10 or less.

The number of ethylene glycol units in the polyethylene glycol may be 2 or more, and is preferably 2 or more and 10 or less.

Examples of the fatty acid optionally having a substituent include linear or branched, monovalent or divalent fatty acids having 4 or more and 24 or less carbon atoms. Examples of the substituent include a hydroxyl group and an acetoxy group. The number of the substituents is preferably 2 or less. Specific examples of the fatty acid optionally having a substituent include lauric acid, oleic acid, caprylic acid, stearic acid, behenic acid, ricinoleic acid, succinic acid, and diacetyl tartaric acid.

In the ester compound of the polyol and the fatty acid, the number of fatty acids bound to one polyol molecule is not particularly limited, and may be appropriately adjusted in accordance with the intended HLB of the emulsifier.

From the viewpoint of suppressing the oxidation of reduced coenzyme Q10, the number of fatty acids bound to one polyol molecule may be, for example, 12 or less, preferably 10 or less, more preferably 7 or less, more preferably 6 or less, more preferably 5 or less, more preferably 3 or less, more preferably 2 or less, more preferably 1.

Specific examples of the ester compound of the polyol and the fatty acid include diglycerol monooleate, monoglycerol monocaprylate, diglycerol monocaprylate, decaglycerol pentaoleate, tetraglycerol pentaoleate, pentaglycerol trioleate, decaglycerol monolaurate, hexaglycerol monocaprylate, hexaglycerol monooleate, pentaglycerol monostearate, tetraglycerol tristearate, decaglycerol monobehenate, mono- and di-glycerol monostearates, monoglycerol monooleate, glycerol monostearate succinate, monoglycerol succinate, glycerol monostearate diacetyl tartrate, propylene glycol monooleate, sorbitan monooleate, sorbitan monostearate, sorbitan tristearate, monoglycerol monolaurate, diglycerol monolaurate, diglycerol monomyristate, tetraglycerol pentastearate, polyoxyethylene sorbitan monooleate, polyoxyethylene sorbitan monolaurate, polyoxyethylene sorbitan monostearate, condensed pentaglycerol ricinoleate, sucrose stearate, sucrose erucate, and sucrose oleate.

In the ester compound of the polyol and the fatty acid, the fatty acid is more preferably an unsaturated fatty acid from the viewpoint of controlling the oxidation of reduced coenzyme Q10. Examples of the unsaturated fatty acid include: crotonic acid, myristoyleic acid, palmitoleic acid, sapienic acid, oleic acid, elaidic acid, vaccenic acid, gadoleic acid, eicosenoic acid, erucic acid, nervonic acid, linoleic acid, eicosadienoic acid, docosadienoic acid, α-linolenic acid, γ-linolenic acid, pinolenic acid, α-eleostearic acid, β-eleostearic acid, mead acid, dihomo-γ-linolenic acid, eicosatrienoic acid, stearidonic acid, arachidonic acid, eicosatetraenoic acid, adrenic acid, bosseopentaenoic acid, eicosapentaenoic acid, osbond acid, clupanodonic acid, tetracosapentaenoic acid, docosahexaenoic acid, nisinic acid, ricinoleic acid, and condensed ricinoleic acid; more preferably oleic acid, condensed ricinoleic acid, linoleic acid, and erucic acid; particularly preferably oleic acid.

From another viewpoint of controlling the oxidation of reduced coenzyme Q10, the ester compound of the polyol and the fatty acid is more preferably a glycerol fatty acid ester or a polyglycerol fatty acid ester that satisfies any of the following:

1) a glycerol fatty acid ester or a polyglycerol fatty acid ester having 1 or more fatty acid residues in which the fatty acid has 8 or less carbon atoms;
2) a polyglycerol fatty acid ester with 2 or more glycerol units having 1 or more fatty acid residues in which the fatty acid has 9 or more and 14 or less carbon atoms; and
3) a polyglycerol fatty acid ester with 5 or more glycerol units having 1 or more fatty acid residues in which the fatty acid has 15 or more and 18 or less carbon atoms.

In these cases, the fatty acid may be a saturated fatty acid or an unsaturated fatty acid.

Specific examples of the ester compound of the polyol and the unsaturated fatty acid include monoglycerol monooleate, monoglycerol mono- and di-oleates, monoglycerol dioleate, mono- and di-glycerol monooleates, diglycerol monooleate, diglycerol mono- and di-oleates, diglycerol dioleate, diglycerol trioleate, triglycerol monooleate, triglycerol dioleate, triglycerol trioleate, triglycerol tetraoleate, tetraglycerol monooleate, tetraglycerol dioleate, tetraglycerol trioleate, tetraglycerol tetraoleate, tetraglycerol pentaoleate, pentaglycerol monooleate, pentaglycerol dioleate, pentaglycerol trioleate, pentaglycerol tetraoleate, pentaglycerol pentaoleate, pentaglycerol hexaoleate, hexaglycerol monooleate, hexaglycerol dioleate, hexaglycerol trioleate, hexaglycerol tetraoleate, hexaglycerol pentaoleate, hexaglycerol hexaoleate, hexaglycerol heptaoleate, decaglycerol monooleate, decaglycerol dioleate, decaglycerol trioleate, decaglycerol tetraoleate, decaglycerol pentaoleate, decaglycerol hexaoleate, decaglycerol heptaoleate, decaglycerol octaoleate, decaglycerol nonaoleate, decaglycerol decaoleate, decaglycerol dodecaoleate, diacetoglycerol monooleate, glycerol monooleate lactate, glycerol monooleate succinate, glycerol monooleate citrate, glycerol monooleate diacetyl tartrate, sucrose oleate, propylene glycol monooleate, sorbitan monooleate, sorbitan dioleate, sorbitan trioleate, polyoxyethylene sorbitan monooleate, 1-palmitoyl-2-oleoylphosphatidylcholine, phosphatidylcholine dilinoleate, monoglycerol monoerucate, monoglycerol mono- and di-erucates, monoglycerol dierucate, mono- and di-glycerol monoerucates, diglycerol monoerucate, diglycerol mono- and di-dierucates, diglycerol dierucate, diglycerol trierucate, triglycerol monoerucate, triglycerol dierucate, triglycerol trierucate, triglycerol tetraerucate, tetraglycerol monoerucate, tetraglycerol dierucate, tetraglycerol trierucate, tetraglycerol tetraerucate, tetraglycerol pentaerucate, pentaglycerol monoerucate, pentaglycerol dierucate, pentaglycerol trierucate, pentaglycerol tetraerucate, pentaglycerol pentaerucate, pentaglycerol hexaerucate, hexaglycerol monoerucate, hexaglycerol dierucate, hexaglycerol trierucate, hexaglycerol tetraerucate, hexaglycerol pentaerucate, hexaglycerol hexaerucate, hexaglycerol heptaerucate, decaglycerol monoerucate, decaglycerol dierucate, decaglycerol trierucate, decaglycerol tetraerucate, decaglycerol pentaerucate, decaglycerol hexaerucate, decaglycerol heptaerucate, decaglycerol octaerucate, decaglycerol nonaerucate, decaglycerol decaerucate, decaglycerol dodecaerucate, diacetoglycerol monoerucate, glycerol monoerucate lactate, glycerol monoerucate succinate, glycerol monoerucate citrate, glycerol monoerucate diacetyl tartrate, sucrose erucate, propylene glycol monoerucate, sorbitan monoerucate, sorbitan dierucate, sorbitan trierucate, condensed monoglycerol ricinoleate, condensed diglycerol ricinoleate, condensed triglycerol ricinoleate, condensed tetraglycerol ricinoleate, condensed pentaglycerol ricinoleate, condensed hexaglycerol ricinoleate, condensed heptaglycerol ricinoleate, and condensed decaglycerol ricinoleate.

Specific examples of the ester compound of the polyol and the unsaturated fatty acid include: preferably monoglycerol monooleate, monoglycerol mono- and di-oleates, monoglycerol dioleate, mono- and di-glycerol monooleates, diglycerol monooleate, diglycerol mono- and di-oleates, diglycerol dioleate, diglycerol trioleate, triglycerol monooleate, triglycerol dioleate, triglycerol trioleate, triglycerol tetraoleate, tetraglycerol monooleate, tetraglycerol dioleate, tetraglycerol trioleate, tetraglycerol tetraoleate, tetraglycerol pentaoleate, pentaglycerol monooleate, pentaglycerol dioleate, pentaglycerol trioleate, pentaglycerol tetraoleate, pentaglycerol pentaoleate, pentaglycerol hexaoleate, hexaglycerol monooleate, hexaglycerol dioleate, hexaglycerol trioleate, hexaglycerol tetraoleate, hexaglycerol pentaoleate, hexaglycerol hexaoleate, hexaglycerol heptaoleate, decaglycerol monooleate, decaglycerol dioleate, decaglycerol trioleate, decaglycerol tetraoleate, decaglycerol pentaoleate, decaglycerol hexaoleate, decaglycerol heptaoleate, decaglycerol octaoleate, decaglycerol nonaoleate, decaglycerol decaoleate, decaglycerol dodecaoleate, diacetoglycerol monooleate, glycerol monooleate lactate, glycerol monooleate succinate, glycerol monooleate citrate, glycerol monooleate diacetyl tartrate, sucrose oleate, propylene glycol monooleate, sorbitan monooleate, sorbitan dioleate, sorbitan trioleate, polyoxyethylene sorbitan monooleate, 1-palmitoyl-2-oleoylphosphatidylcholine; particularly preferably diglycerol monooleate.

From the viewpoint of suppressing the oxidation of reduced coenzyme Q10, a polyoxyethylene sorbitan fatty acid ester is also preferable as the ester compound of the polyol and the fatty acid.

Specific examples of the polyoxyethylene sorbitan fatty acid ester include polyoxyethylene sorbitan monooleate, polyoxyethylene sorbitan monolaurate, and polyoxyethylene sorbitan monostearate.

When the solid composition containing the emulsifier is used in combination with an oily component (for example, vegetable oil, essential oil, animal oil or fat, fish oil, a lipid-soluble active component, or the like), the emulsifier contained in the solid composition preferably has an HLB of less than 10.0, more preferably an HLB of less than 8.0, particular preferably an HLB of less than 6.0. The use of the ester composed of the polyol and the unsaturated fatty acid and having an HLB of less than 6.0 as the emulsifier allows the reduced coenzyme Q10 in the solid composition to be stably stored even under conditions of a relative humidity of less than 50%, i.e., conditions under which pharmaceutical products and foods are often stored, and is therefore preferable.

Specific examples of the ester composed of the polyol and the unsaturated fatty acid and having an HLB of less than 6.0 include monoglycerol monooleate, monoglycerol mono- and di-oleates, monoglycerol dioleate, mono- and di-glycerol monooleates, tetraglycerol pentaoleate, hexaglycerol pentaoleate, decaglycerol pentaoleate, decaglycerol decaoleate, sorbitan monooleate, sorbitan trioleate, propylene glycol monooleate, monoglycerol monooleate citrate, sucrose oleate, decaglycerol erucate, and sucrose erucate.

Examples of the lecithin include soybean lecithin, egg yolk lecithin, and enzymolytic soybean lecithin.

The emulsifier to be used is particularly preferably an emulsifier acceptable for foods, cosmetics, and/or pharmaceutical products.

<Antioxidant>

Herein, the antioxidant is not limited to any kind. Two or more kinds of antioxidants may be used in combination. The antioxidant is preferably solid at ordinary temperature, and specific examples include one or more selected from the group consisting of ascorbic acid, ascorbic acid salts, erythorbic acid, and erythorbic acid salts.

The counterion of each of the ascorbic acid salt and the erythorbic acid salt is not limited, and the ascorbic acid salt and the erythorbic acid salt may be independently one or more metal salts selected from the group consisting of sodium salts, potassium salts, calcium salts, and magnesium salts.

The antioxidant to be used is particularly preferably an antioxidant acceptable for foods or pharmaceutical products. The antioxidant is preferably an ascorbic acid salt, particularly preferably one or more selected from the group consisting of sodium ascorbate and calcium ascorbate.

<Binder>

The solid composition disclosed herein preferably further contains a binder. The binder can be used for binding components including the co-crystal of reduced coenzyme Q10, the emulsifier, and the antioxidant to form a particulate solid composition.

The binder is not limited to any kind. Two or more kinds of binders may be used in combination. Specific examples of the binder include one or more selected from the group consisting of celluloses and starches.

Examples of the celluloses include hydroxypropyl cellulose, hydroxypropylmethyl cellulose, hydroxymethyl cellulose, carboxymethyl cellulose, crystalline cellulose, powdered cellulose, methyl cellulose, ethyl cellulose, and salts thereof. The binder is particularly preferably one or more selected from the group consisting of hydroxypropyl cellulose, hydroxypropylmethyl cellulose, and sodium carboxymethyl cellulose.

Examples of the starches include wheat starch, potato starch, sweet potato starch, corn starch, dextrin, hydroxypropyl starch, starch acetate, oxidized starch, octenyl succinate starch and salts thereof, and partially pregelatinized starch.

The binder to be used is particularly preferably a binder acceptable for foods or pharmaceutical products.

<Solid Composition>

One or more embodiments of the present invention relate to a solid composition containing the co-crystal of reduced coenzyme Q10, the emulsifier, and the antioxidant.

Even when stored under conditions involving contact with air, the solid composition according to the present embodiment allows to suppress the decrease of the reduced coenzyme Q10 due to oxidation and is thus suitable for storage for a long time, for example, a period of three months or more. The storage temperature for the solid composition is, for example, 0°C or more and 45°C or less, preferably 0°C or more and 40°C or less, more preferably 5°C or more and 25°C or less. Under the condition of a temperature of 25°C or less, the solid composition according to the present embodiment can maintain the reduced coenzyme Q10 in an amount of 90% by weight or more of the initial amount for a period as long as 20 months or more. In order to provide the solid composition in the above-described form at a reasonable price, it is preferable that the reduced coenzyme Q10 can be maintained in an amount of 90% by weight or more, more preferably 95% by weight or more, most preferably 97% by weight or more, of the initial amount for three months under conditions of 25°C together with silica gel in a hermetically sealed system, or for one month under conditions of 40°C together with silica gel in a hermetically sealed system.

When the solid composition is stored under more severe conditions, such as the accelerated testing conditions, for example, under the environment of 40°C and 75%RH in an open state for 30 days, a residual rate of the reduced coenzyme Q10 (the QH residual rate) is typically 93% or more, and it is preferably 94% or more, 95% or more, 96% or more, or 97% or more.

The solid composition according to the present embodiment is a composition that is solid at 25°C. The solid composition according to the present embodiment may have any shape, such as a particulate, powdery, or flaky shape, and preferably has a particulate shape. The particulate shape may be any shape formed by binding primary particles of a material containing the co-crystal of reduced coenzyme Q10, the emulsifier, and the antioxidant by granulation, and encompasses a granular shape. The particulate solid composition is not limited to any dimensions, and can be, for example, granules having a longest diameter of 0.20 mm or more and 2.0 mm or less.

The solid composition according to the present embodiment can be produced at a high yield and has good handling properties even when the emulsifier used is a liquid, sol, gel, or soft solid. For example, the solid composition produced by high-shear granulation exhibits a pass rate of 50% or more, preferably 60% or more, more preferably 70% or more, more preferably 80% or more, particularly preferably 90% or more, with respect to a mesh having an opening of 710 μm. In this regard, the size of the mesh opening is generally adjusted in accordance with an assumed particle diameter. For granules obtained by extrusion granulation, a mesh having an opening of 3.0 mm or the like can be used.

In addition, the solid composition according to the present embodiment contains the co-crystal of reduced coenzyme Q10 in a crystalline form. The crystallinity of the co-crystal (the ratio of the amount of the reduced coenzyme Q10 present as the co-crystal to the total amount of the reduced coenzyme Q10 contained in the solid composition) is typically 50% or more, preferably 70% or more, more preferably 90% or more, particularly preferably 95% or more.

The crystallinity of the co-crystal contained in the solid composition can be determined by measuring the heat of melting of the crystal by DSC analysis, then calculating the crystallinity in accordance with the formula described below using the measured heat of melting and the theoretical heat of melting determined from the amount of the co-crystal contained in the solid composition.

$$\text{Crystallinity (\%)} = (\text{Measured heat of melting/Theoretical heat of melting}) \times 100$$

Additionally, in the solid composition according to the present embodiment, the co-crystal of reduced coenzyme Q10 does not need to be completely coated with a gas-barrier material. Accordingly, the solid composition does not have its oxidation stability compromised by coating breakage even under pressure and can therefore be applied to a wide variety of formulation forms, such as compression tablets.

In the solid composition according to the present embodiment, the ratio of each component can be adjusted so that the oxidation of the reduced coenzyme Q10 can be suppressed.

The solid composition according to the present embodiment more preferably contains the antioxidant, for example, at 1 part by weight or more and 9900 parts by weight or less with respect to 100 parts by weight of the co-crystal of reduced coenzyme Q10. The lower limit of the amount of the antioxidant with respect to 100 parts by weight of the co-crystal of reduced coenzyme Q10 is preferably 20 parts by weight or more, more preferably 50 parts by weight or more, more preferably 80 parts by weight or more. The upper limit of the amount of the antioxidant with respect to 100 parts by weight of the co-crystal of reduced coenzyme Q10 is preferably 5000 parts by weight or less, more preferably 1000 parts by weight or less, more preferably 500 parts by weight or less, more preferably 200 parts by weight or less, more preferably 120 parts by weight or less.

The solid composition according to the present embodiment more preferably contains the emulsifier, for example, at 1 part by weight or more and 9900 parts by weight or less with respect to 100 parts by weight of the co-crystal of reduced coenzyme Q10. The lower limit of the amount of the emulsifier with respect to 100 parts by weight of the co-crystal of reduced coenzyme Q10 is preferably 3 parts by weight or more, more preferably 5 parts by weight or more, more preferably 8 parts by weight or more. The upper limit of the amount of the emulsifier with respect to 100 parts by weight of the co-crystal of reduced coenzyme Q10 is preferably 5000 parts by weight or less, more preferably 1000 parts by weight or less, more preferably 500 parts by weight or less, more preferably 200 parts by weight or less, more preferably 150 parts by weight or less, more preferably 100 parts by weight or less, more preferably 50 parts by weight or less, more preferably 30 parts by weight or less.

The lower limit of the amount of the co-crystal of reduced coenzyme Q10 contained in the solid composition according to the present embodiment is, for example, 1% by weight or more, preferably 10% by weight or more, more preferably 20% by weight or more, more preferably 30% by weight or more. The upper limit of the amount of the co-crystal of reduced coenzyme Q10 is, for example, 90% by weight or less, preferably 80% by weight or less, more preferably 70% by weight or less, more preferably 60% by weight or less. In the solid composition according to the present embodiment, the co-crystal of reduced coenzyme Q10 does not need to be completely coated with a gas-barrier material. Thus, a larger amount of the co-crystal, consequently a larger amount of the reduced coenzyme Q10 can be contained, and the amount of the co-crystal of reduced coenzyme Q10 can be, for example, 45% or more, 50% or more, 55% or more, 60% or more, 70% or more, or 80% or more.

The lower limit of the amount of the antioxidant contained in the solid composition according to the present embodiment is, for example, 1% by weight or more, preferably 10% by weight or more, more preferably 20% by weight or more, more preferably 30% by weight or more. The upper limit of the amount of the antioxidant is, for example, 99% by weight or less, preferably 90% by weight or less, preferably 80% by weight or less, more preferably 70% by weight or less, more preferably 60% by weight or less.

The amount of the emulsifier contained in the solid composition according to the present embodiment is not particularly limited. The lower limit of the amount of the emulsifier is, for example, 0.5% by weight or more, preferably 1% by weight or more, more preferably 3% by weight or more. The upper limit of the amount of the emulsifier is, for example, 99% by weight or less, preferably 50% by weight or less, more preferably 30% by weight or less, more preferably 25% by weight or less, more preferably 20% by weight or less, more preferably 15% by weight or less, more preferably 10% by weight or less. When the emulsifier used is in a form other than powder or flakes, for example, one or more forms selected from the group consisting of liquid, sol, gel, and soft solid, it is conventionally difficult to obtain the solid composition having good handling properties at a high yield. However, when the amount of the emulsifier contained in the solid composition according to the present embodiment is, for example, 50% by weight or less, preferably 30% by weight or less, more preferably 25% by weight or less, it is possible to obtain the solid composition having good handling properties at a high yield.

When the solid composition according to the present embodiment contains the binder, the lower limit of the amount of the binder contained in the solid composition is, for example, 1% by weight or more, preferably 5% by weight or more, more preferably 8% by weight or more. The upper limit of the amount of the binder is, for example, 20% by weight or less, preferably 15% by weight or less, more preferably 10% by weight.

The solid composition according to the present embodiment more preferably contains the binder, for example, at an amount of 1 part by weight or more, preferably 3 parts by weight or more, more preferably 5 parts by weight or more, more preferably 8 parts by weight or more, with respect to 100 parts by weight of the co-crystal of reduced coenzyme Q10. In addition, the solid composition contains the antioxidant, for example, at an amount of 200 parts by weight or less, preferably 150 parts by weight or less, more preferably 100 parts by weight or less, more preferably 50 parts by weight or less, still more preferably 30 parts by weight or less, with respect to 100 parts by weight of the co-crystal of reduced coenzyme Q10.

The solid composition according to the present embodiment may further contain another component such as an excipient. Another component is preferably a component acceptable for foods or pharmaceutical products.

The solid composition according to the present embodiment is preferably free of a carbonate containing a sodium cation and/or a calcium cation. Examples of the carbonate containing a sodium cation include sodium carbonate and hydrates thereof (for example, monohydrates, heptahydrates, decahydrates, and the like) and sodium bicarbonate. Examples of the carbonate containing a calcium cation include calcium carbonate and hydrates thereof (for example, 0.65-hydrates, monohydrates, 1.5-hydrates, hexahydrates, and the like).

The solid composition according to the present embodiment is preferably free of porous calcium silicate. Examples of the porous calcium silicate include a calcium silicate having an average particle diameter of 18 to 32 μm, a loose bulk density of 0.07 to 0.15 g/mL, and an oil absorption rate of 300 to 550 mL/100 g.

<Method for Producing Particulate Solid Composition>

Another one or more embodiments of the present invention relate to a method for producing a particulate solid composition containing a co-crystal of reduced coenzyme Q10, an emulsifier, an antioxidant, and a binder, the method comprising granulating a mixture containing the co-crystal of reduced coenzyme Q10, the emulsifier, the antioxidant, the binder, and a liquid binder.

In the method according to the present embodiment, the co-crystal of reduced coenzyme Q10 and a solid antioxidant are granulated in the presence of the emulsifier (which is preferably a liquid, sol, gel, or soft solid emulsifier), thereby producing the particulate solid composition characterized by having a high storage stability in a low cost and simple manner.

A granulation method can be appropriately selected. For example, an extrusion granulation method, high-shear granulation method, tumbling granulation method (rotation granulation method), dry granulation method, compression granulation method, powder joining method (particle composing, surface treatment and spheronization of dry powder by high shear), fluidized-bed granulation method, coacervation method, spray-dry method, coldspray method, evaporation method, or in situ gelation coating method (orifice method) can be selected. The granulation method to be performed is preferably a granulation method with pressure (for example, extrusion granulation method, high-shear granulation method, tumbling granulation method, dry granulation method, compression granulation method, or powder joining method), or a fluidized-bed granulation method, more preferably an extrusion granulation method, high-shear granulation method, or fluidized-bed granulation method, particularly preferably an extrusion granulation method or high-shear granulation method.

In the method according to the present embodiment, the reduced coenzyme Q10 crystal is not completely dissolved or melted during a granulation process, and typically maintains its crystalline state during mixing and granulation.

The components other than a liquid binder in the mixture to be subjected to granulation can be a powdery mixture. The properties of each of the components other than the liquid binder in the mixture are as described for each of the components contained in a solid composition according to one or more embodiments of the present invention.

Examples of the liquid binder include water and ethanol. In addition, the liquid binder may be prepared by dissolving the above-described binder in a liquid such as water or ethanol. The liquid binder is particularly preferably water.

The granulation may be carried out under appropriate conditions to produce a particulate solid composition having a size suitable for the intended use.

The method according to the present embodiment preferably further includes a drying step of drying the granulated particulate solid composition to remove volatile components derived from the raw material components and the liquid binder. Additionally, if desired, a particulate solid composition having a desired particle diameter can be separated and collected by sieving or the like.

Examples

One or more embodiments of the present invention will be further specifically described with reference to the following Examples, but the present invention is not limited to these Examples. It should be noted that the HPLC measurement conditions in Examples and Comparative Examples are as described below.

(Method for Evaluating Oxidation Stability)

The weight ratio of the reduced coenzyme Q10 to the total coenzyme Q10 (i.e., reduced coenzyme Q10/(oxidized coenzyme Q10 + reduced coenzyme Q10)) is defined as a "QH ratio". The QH ratio was determined by the following HPLC analysis. For the evaluation of oxidation stability, a rate of the change in the QH ratio at the end of the evaluation from that at the beginning of the evaluation was defined as "QH residual rate", and the QH residual rate determined by the following formula was used as a measure of oxidation stability.

QH residual rate (%) = 100 × QH ratio at the end of the evaluation/QH ratio at the beginning of the evaluation

(HPLC Measurement Conditions)

Column: SYMMETRY $C_{18}$ (manufactured by Waters Corporation); 250 mm (in length) and 4.6 mm (in inner diameter)
Mobile phase: $C_2H_5OH$:$CH_3OH$ = 4:3 (v:v)
Detection wavelength: 210 nm
Flow rate: 1 mL/min

(Example of Co-crystal Preparation)

The co-crystal of reduced coenzyme Q10 with nicotinamide (hereafter, it may be denoted as the "reduced coenzyme Q10/nicotinamide co-crystal) was prepared in the manner described below in accordance with the Examples of WO 2023/120552.

4.33 g of QH Form I crystal and 1.22 g of nicotinamide were added to 7.85 g of ethanol, and the temperature was raised to 50°C to completely dissolve the QH Form I crystal and nicotinamide. The solution was cooled and the resulting white slurry was dried under reduced pressure to obtain a co-crystal of QH and nicotinamide. The melting point of the resulting co-crystal was measured using a differential scanning calorimeter (DSC200, manufactured by Hitachi, the temperature increase rate: 1°C/min). As a result, a peak was observed at a temperature different from the melting point (48°C) of the QH Form I crystal and the melting point (127°C) of nicotinamide used as the raw materials. Reduced coenzyme Q10 (trade name: Kaneka QH) manufactured by Kaneka Corporation was used as the reduced coenzyme Q10 Form I crystal (QH Form I crystal).

(Comparative Example 1)

A reduced coenzyme Q10 Form I crystal in an amount of 1.0 g, 1.0 g of sodium ascorbate, 0.2 g of hydroxypropyl cellulose, and 0.2 g of diglycerol monooleate (SUNSOFT Q-17D, manufactured by Taiyo Kagaku Co., Ltd.; having an HLB value of 7.5) were mixed, and 0.09 mL of water was added. The resulting mixture was kneaded. The kneaded material obtained was extrusion-granulated using a 1.2 mm Φ screen, and dried to obtain a particulate solid composition (granulated product). The resulting particulate solid composition in an amount of 1 g was stored under the environment of 25°C and 60%RH in an open state for 30 days to evaluate the QH residual rate.
The QH residual rate was 52.0%. That is, the particulate solid composition prepared with the use of the reduced coenzyme Q10 Form I crystal was insufficient in terms of oxidation stability of the reduced coenzyme Q10.

(Comparative Example 2)

The reduced coenzyme Q10/nicotinamide co-crystal in an amount of 1 g each was weighed into two 30-ml glass vials (under the operating environment of 28°C and 50%RH). One of the vials was kept in an open state, the other vial was hermetically sealed with a screw cap (sealed state), these vials were stored under the environment of 40°C and 75%RH for 30 days, and the residual rate of reduced coenzyme Q10 was evaluated.

(Example 1)

The reduced coenzyme Q10/nicotinamide co-crystal in an amount of 1 g, 1 g of sodium ascorbate, 0.2 g of hydroxypropyl cellulose, and 0.2 g of diglycerol monooleate (SUNSOFT Q-17D; having an HLB value of 7.5) were mixed, and water was added. The resulting mixture was kneaded. The kneaded material obtained was extrusion-granulated using a 1.2 mm Φ screen, and dried to obtain a particulate solid composition. In the same manner as in Comparative Example 2, the resulting particulate solid composition in an amount of 1 g each was weighed into two 30-ml glass vials, the glass vials were stored under the environment of 40°C and 75%RH in an open state and in a hermetically sealed state for 30 days, and the residual rate of the reduced coenzyme Q10 was evaluated.
The results of Comparative Example 2 and of Example 1 are shown in Table 1.

[Table 1]

| QH residual rate after storage at 40°C and 75%RH for 30 days | | | |
|---|---|---|---|
| | Stored sample | QH residual rate (%) | |
| | | Open state | Sealed state |
| Comp. Ex. 2 | Reduced coenzyme Q10/nicotinamide co-crystal | 90.9 | 92.9 |
| Ex. 1 | Particulate solid composition containing reduced coenzyme Q10/nicotinamide co-crystal | 97.6 | 100 |

The results demonstrate that the reduced coenzyme Q10/nicotinamide co-crystal has stability that is higher than that of the reduced coenzyme Q10 Form I crystal but such co-crystal is required to be further stabilized to be applied to a wide variety of dosage forms. The results also demonstrate that the solid composition of the present invention can enhance the oxidation stability to a significant extent.

Although the present embodiments are described in detail above, the specific configuration is not limited to these embodiments, and the present disclosure encompasses any design modification within the scope which does not depart from the spirit of the present disclosure.

All publications, patents, and patent applications cited herein are incorporated herein by reference in their entirety.

**Claims**

1. A solid composition containing a co-crystal of reduced coenzyme Q10, an emulsifier, and an antioxidant.

2. The solid composition according to claim 1, wherein the emulsifier is in the form of liquid, sol, gel, or soft solid at 50°C.

3. The solid composition according to claim 1 or 2, wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

4. The solid composition according to any one of claims 1 to 3, wherein an amount of the emulsifier contained in the solid composition is 1% by weight or more and 99% by weight or less.

5. The solid composition according to any one of claims 1 to 4, wherein the antioxidant is one or more selected from the group consisting of ascorbic acid, ascorbic acid salts, erythorbic acid, and erythorbic acid salts.

6. The solid composition according to any one of claims 1 to 5, wherein the co-crystal of reduced coenzyme Q10 is a co-crystal of reduced coenzyme Q10 with benzoic acids or a co-crystal of reduced coenzyme Q10 with nicotinamide.

7. The solid composition according to any one of claims 1 to 6, wherein 70% by weight or more of the reduced coenzyme Q10 is contained in the solid composition in the form of the co-crystal of reduced coenzyme Q10.

8. The solid composition according to any one of claims 1 to 7, wherein a residual rate of the reduced coenzyme Q10 after the solid composition is stored under the environment of 40°C and 75%RH in an open state for 30 days is 93% or more.

9. The solid composition according to any one of claims 1 to 8, further containing a binder.

10. The solid composition according to claim 9, wherein the solid composition is a particulate solid composition.

11. The solid composition according to any one of claims 1 to 10, wherein the solid composition is free of a carbonate containing a sodium cation and/or a calcium cation.

12. The solid composition according to any one of claims 1 to 11, wherein the solid composition is free of porous calcium silicate.

13. A method for producing a particulate solid composition containing a co-crystal of reduced coenzyme Q10, an emulsifier, an antioxidant, and a binder,
the method comprising granulating a mixture containing the co-crystal of reduced coenzyme Q10, the emulsifier, the antioxidant, the binder, and a liquid binder.

14. The method according to claim 13, wherein the granulating is performed by an extrusion granulation method, a high-shear granulation method, or a fluidized-bed granulation method.

## INTERNATIONAL SEARCH REPORT

| International application No. |
|---|
| **PCT/JP2024/030631** |

### A. CLASSIFICATION OF SUBJECT MATTER

*A61K 31/122*(2006.01)i; *A61K 9/14*(2006.01)i; *A61K 31/19*(2006.01)i; *A61K 31/455*(2006.01)i; *A61K 47/10*(2017.01)i; *A61K 47/14*(2017.01)i; *A61K 47/22*(2006.01)i; *A61K 47/26*(2006.01)i; *A61K 47/34*(2017.01)i
FI: A61K31/122; A61K47/10; A61K47/34; A61K47/26; A61K47/14; A61K47/22; A61K31/455; A61K31/19; A61K9/14

According to International Patent Classification (IPC) or to both national classification and IPC

### B. FIELDS SEARCHED

Minimum documentation searched (classification system followed by classification symbols)

A61K31/122; A61K9/14; A61K31/19; A61K31/455; A61K47/10; A61K47/14; A61K47/22; A61K47/26; A61K47/34

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2021-514370 A (CENTER FOR INTELLIGENT RESEARCH IN CRYSTAL ENGINEERING, S. L.) 10 June 2021 (2021-06-10) claims (e.g. claim 14 citing 1-13), paragraphs [0086], [0087], examples, etc. | 1-14 |
| Y | WO 2019/167663 A1 (PETROEUROASIA CO., LTD.) 06 September 2019 (2019-09-06) claims (claim 4, etc.), table 1, paragraphs [0020], [0039], [0057], etc., examples | 1-14 |
| Y | JP 2016-520037 A (ZHEJIANG MEDICINE CO., LTD. XINCHANG PHARMACEUTICA FACTORY) 11 July 2016 (2016-07-11) claims (claim 9 etc.), paragraphs [0013], [0028], examples | 1-14 |
| Y | JP 2010-126492 A (KABUSHIKI KAISHA CYCLOCHEM) 10 June 2010 (2010-06-10) claims, examples | 1-14 |
| Y | WO 2008/129980 A1 (KANEKA CORPORATION) 30 October 2008 (2008-10-30) paragraphs [0041], [0042] | 1-14 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **09 October 2024** | **22 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
|---|
| **PCT/JP2024/030631** |

| C. | DOCUMENTS CONSIDERED TO BE RELEVANT | |
|---|---|---|
| Category* | Citation of document, with indication, where appropriate, of the relevant passages | Relevant to claim No. |
| Y | WO 2007/148798 A1 (KANEKA CORPORATION) 27 December 2007 (2007-12-27) paragraphs [0032], [0033] | 1-14 |
| Y | WO 2007/125915 A1 (KANEKA CORPORATION) 08 November 2007 (2007-11-08) paragraph [0025] | 1-14 |

Form PCT/ISA/210 (second sheet) (July 2022)

## INTERNATIONAL SEARCH REPORT
### Information on patent family members

| | International application No. |
| --- | --- |
| | **PCT/JP2024/030631** |

| Patent document cited in search report | | | Publication date (day/month/year) | Patent family member(s) | | | Publication date (day/month/year) |
| --- | --- | --- | --- | --- | --- | --- | --- |
| JP | 2021-514370 | A | 10 June 2021 | WO 2019/162429 A1 claims, description, page 18, lines 8-14, examples, etc. US 2020/0385327 A1 EP 3755683 A1 CN 112041294 A | | | |
| WO | 2019/167663 | A1 | 06 September 2019 | US 2021/0046020 A1 claims, table 1, paragraphs [0030], [0062], [0080], etc., examples EP 3760196 A1 | | | |
| JP | 2016-520037 | A | 11 July 2016 | US 2016/0101053 A1 claims, paragraphs [0018], [0033], examples WO 2014/173174 A1 EP 2995605 A1 CN 104119209 A | | | |
| JP | 2010-126492 | A | 10 June 2010 | (Family: none) | | | |
| WO | 2008/129980 | A1 | 30 October 2008 | US 2010/0092560 A1 paragraphs [0044], [0045] | | | |
| WO | 2007/148798 | A1 | 27 December 2007 | US 2008/0026063 A1 paragraphs [0061], [0062] EP 2039353 A1 KR 10-2009-0034901 A CN 101472573 A | | | |
| WO | 2007/125915 | A1 | 08 November 2007 | US 2009/0186009 A1 paragraph [0032] | | | |

Form PCT/ISA/210 (patent family annex) (July 2022)

**REFERENCES CITED IN THE DESCRIPTION**

**Patent documents cited in the description**

- WO 2006075502 A
- WO 2008129980 A
- WO 2012176842 A
- WO 2015122531 A
- CN 113024362 A

- JP 2010189337 A
- JP S60221078 A
- JP 2023148348 A
- WO 2023120552 A